# EUROPEAN PATENT SPECIFICATION

(11) **EP 1 753 381 B1**
(45) Date of publication and mention of the grant of the patent: **17.10.2012**
(21) Application number: 04736711.5
(22) Date of filing: 11.06.2004
(51) Int. Cl.: A61F 13/15, A61L 15/46

(54) **AN ABSORBENT ARTICLE CONTAINING FRAGRANCE**
SAUGFÄHIGER ARTIKEL MIT EINEM DUFTSTOFF
ARTICLE ABSORBANT A PARFUM

(43) Date of publication of application: 21.02.2007
(73) Proprietor: SCA Hygiene Products AB, 405 03 Göteborg (SE)
(72) Inventor: ALBINO, Robert, S-435 43 Pixbo (SE); LIPSCHUTZ, Oscar, Port Melbourne, VIC 3207 (AU)
(74) Representative: Andersson, Per Rune
(86) International application number: PCT/SE2004/000911
(87) International publication number: WO 2005/120413

(56) References cited:
- EP-A2- 1 090 616
- WO-A1-93/09818
- WO-A1-94/22500
- US-A- 4 186 743
- US-A- 5 591 146
- US-A- 5 733 272
- US-A- 5 769 833
- US-A- 5 951 534
- US-A1- 2004 127 866
- US-B1- 6 369 290
- US-B1- 6 746 418

## Description

### TECHNICAL FIELD

The present invention relates to an absorbent article such as a sanitary towel, a panty liner, a diaper or an incontinence pad comprising a liquid-impermeable or at least liquid-blocking surface layer and also an absorbent body with a liquid-permeable surface, or a separate liquid-permeable surface layer, a fragrance means being arranged on one of the surfaces or the surface layers.

### BACKGROUND ART

The handling and use of absorbent articles such as diapers, incontinence pads, sanitary towels and panty liners intended for the absorption of body fluids can often feel unhygienic on account of the rather unpleasant odour a used absorbent article can give off. In order to increase the impression of cleanness and freshness and in order to counteract bad odour in absorbent articles of this kind, it has therefore been proposed to provide them with additives in the form of various types of aromatic substances, such as perfumes. However, some users of absorbent hygiene articles do not want the article to smell of perfume. Moreover, certain people are hypersensitive or allergic to perfumes and for this reason wish to avoid products with such additives.

It is not appropriate either for the article to emit a perfume fragrance when it is stored, for example in a handbag or in a bathroom cabinet. Fragrances which are a pleasant and fresh experience in one situation can appear less suitable in another. For example, it is perhaps not always appreciated if the entire contents of a handbag smell of perfume. Correspondingly, it may of course be appropriate not to release perfume fragrance in storage spaces such as storerooms, shops and bathrooms. Moreover, the fragrance ages and fades over the time it is exposed to the environment.

US 5,769,833 describes a diaper with perfume zones arranged on the outside of a backing layer. The perfume zones comprise a binder with perfume-emitting means and are covered by detachable strips. When the strips are torn off, the aromatic substances in the perfume zones are released. The strips can be torn off at different times, for example in connection with the article being put on, while the article is being worn, or when the article is taken off after use.

US-A-595 1534 describes a diaper having one or more touch-sensitive fragrance members positioned thereon.

Although it is advantageous that the user can activate the perfume zones personally if and when desired, the disposal of the detachable strips involves a problem for the user. Furthermore, there is a risk of the strips being removed by mistake by becoming caught on another object. For example, the strips may already be torn off by accident when the article is taken out of its packaging, or by, during use, catching on a garment worn outside the absorbent article. Moreover, the application of loose material strips involves a price-raising operation in the manufacture of the absorbent article and increases the consumption of material, which is of course an especially negative feature for articles which are intended to be thrown away when they have been used once.

WO 93/09818 also describes an absorbent article such as a dressing or a sanitary towel with a fragrance-emitting area which is activated by detaching a separate protective layer.

One object of the present invention is to provide an absorbent article which emits perfume fragrance only when this is desirable.

Another object is to offer an absorbent article comprising an improved activatable means for fragrance emission.

### DISCLOSURE OF INVENTION

By way of the present invention, an absorbent article of the kind referred to in the introduction has been produced.

An absorbent article of the kind referred to in the introduction, comprising a fragrance means which is arranged on a surface of the article, is characterized mainly in that the fragrance means has an active, fragrant form and an inactive, non-fragrant form and is arranged on the surface of the article in the inactive form and is activatable by mechanical action such as rubbing or scraping.

According to a preferred embodiment, the fragrance means is arranged on the liquid-blocking surface of the article. Such an embodiment means that the risk of contact between the body of the user and the fragrance means is minimal. Fragrance means can of course be arranged both on the liquid-blocking surface and on the liquid-permeable surface.

One advantage of, according to the invention, arranging the fragrance means so that it is activatable without separate loose parts having to be handled and disposed of is of course that it is possible to activate the fragrance means when so desired. The fragrance means can therefore be activated at any time in connection with and/or during use of the absorbent article. For example, the fragrance means can be fully or partly activated before the article is placed adjacent to the body of the user or in connection with the removal of the used article. It is also possible to activate the fragrance means one or more times during use, that is to say when the article is being worn adjacent to the body. By virtue of it being possible to activate the fragrance means only in part, the user can himself/herself determine how much fragrance is to be emitted and on how many occasions.

Absorbent articles intended to be worn in the crotch of a user and to be arranged to absorb body discharges such as urine, motions and menstrual blood usually have an elongate shape with a first end portion, a second end portion and an intermediate portion located between the two end portions. It may then be advantageous if the fragrance means is arranged on the liquid-blocking surface layer in at least one end portion. Such an embodiment makes it easy to reach the fragrance means even when the absorbent article is being worn adjacent to the body.

Absorbent articles such as sanitary towels, incontinence pads for mildly incontinent people and panty liners are usually attached inside the briefs of the user by means of an adhesive attachment element. Such attachment elements are found in the form of self-adhesive glued surfaces which may cover all or parts of the outside of the liquid-impermeable surface layer. It is also common to use other types of attachment element such as friction surfaces, hook and loop surfaces and clips. According to one embodiment of the invention, a preferably adhesive attachment element is arranged on the liquid-impermeable surface layer in the end portions of the article, the fragrance means being arranged in an area free of attachment elements in the intermediate portion of the article. Such an embodiment affords secure attachment of the end portions of the article but allows a certain mobility of the intermediate portion. This is advantageous as it increases the capacity of the intermediate portion for adapting to the shape of the body of the user. This embodiment is especially suitable for absorbent articles with a raised portion arranged on the liquid-permeable surface as it makes it possible to keep the raised portion in continuous contact with the body during body movements as well.

There are various ways of arranging a fragrance means so that it can be activated by scraping or rubbing. For example, the fragrance means can be encapsulated in microcapsules, the walls of which can be broken by mechanical action such as friction or pressure. According to the present invention a fragrance means which is activatable by scraping or rubbing is provided by virtue of the fragrance means in its inactive form being covered by a scrape-off coating or a breakable membrane. When the coating is removed or the membrane is broken, the fragrance means, which may be, for example, a fragrant oil arranged on a porous carrier or in a gel, Is exposed.

The fragrance means can also be applied to a part surface of the liquid-blocking surface layer and be covered by a material layer which is attached to the liquid-impermeable surface layer only outside the part surface provided with the fragrance means, the material layer being movable in relation to the fragrance means. In such an embodiment, the material layer therefore has attachments in areas located around the part surface provided with fragrance means on the liquid-impermeable surface layer, while the part surface provided with fragrance means is free of attachments to the material layer. In this way, it is possible by a rubbing movement to move the material layer in relation to the fragrance means and the liquid-blocking surface layer, so that friction forces which can activate the fragrance means arise. It is therefore appropriate to select a material for the material layer which has high friction. Such materials are materials with an uneven surface, such as coarse-fibred non-woven materials, embossed non-woven materials or plastic films, or coarse-fibred and/or embossed plastic or textile nets.

It is suitable if the separate material layer is permeable to the aromatic substance in the fragrance means, for example by virtue of being porous or perforated. However, it is possible to use impermeable materials and Instead to have the aromatic substance escape to the environment in a gap between the liquid-impermeable surface layer and the separate material layer.

One advantage of the fragrance means being covered by a separate material layer is that it is thus possible to avoid direct contact between the hands of the user and the fragrance means when the fragrance means is activated. According to another embodiment of the invention, the fragrance means comprises a carrier in the form of a material tab attached to the surface of the article. The material tab can then contain microcapsules with aromatic substance which is activated when the material tab is rubbed. In this connection, the material tab is suitably attached permanently to the liquid-impermeable surface layer along one edge and attached detachably to the liquid-impermeable surface layer along another edge so that it is held in contact with the surface of the article before activation of the fragrance means. When the fragrance means is to be activated, the detachable attachment is freed and the material tab is folded out from the surface of the article around the permanent attachment so that the fragrance means becomes accessible for activation. During use of the absorbent article, the material tab can either be re-attached to the liquid-impermeable surface layer or left freely movable in relation to it. In this embodiment as well, it is suitable, but not necessary, for the material tab to be permeable to the aromatic substances of the fragrance means.

According to another embodiment, the article has side flaps which are arranged at the side edges of the article and have such a shape and size that they can be folded around the leg edges on a pair of briefs and attached in the folded position on the outside of the briefs, the fragrance means being arranged on the side flaps. It is then possible to arrange the fragrance means either on the liquid-blocking surface layer or on the opposite surface. In the first case, it is necessary to ensure that the aromatic substance in the fragrance means can escape through the side flaps when these are attached to the crotch of briefs. In the second case, the fragrance means will face outwards and is therefore exposed to the environment during use.

In order for it to be possible for the user to know rapidly and easily where the fragrance means is located, it may be suitable if the positioning of the fragrance means is marked by a visually identifiable indication. Such a marking can be in the form of a decorative pattern, a differing colour, a differing material, text, a logo or the like. It is also possible to use touch-detectable indicators such as embossed surfaces, differing material or the like. Such an embodiment facilitates activation of the fragrance means when the absorbent article is being worn adjacent to the body.

According to a further embodiment, the fragrance means in the inactive form is covered by a partly detachable protective layer. The protective layer is suitably attached to the article along an edge portion of the protective layer and is foldable around the attachment and can thus occupy a non-folded protective position and a folded-away exposure position in which the fragrance means becomes accessible for activation. The article preferably also comprises means for fixing the protective layer in the exposure position. Such a means can be an adhesive or some type of mechanical attachment element such as touch and close surfaces. Depending on where the protective layer is positioned on the surface of the article, it may be possible to utilize a fastening element, for example a glue fastening element on the liquid-blocking layer on a sanitary towel, as fixing element for the protective layer.

Suitable fragrances for use in the fragrance means according to the invention are those associated with cleanness and freshness and include various types of perfume fragrance, lavender fragrance, "washing detergent fragrance" and the like.

### DESCRIPTION OF FIGURES

The invention will be described in greater detail below with reference to the figures shown in accompanying drawings, in which:
- Figure 1: shows a sanitary towel with a fragrance means according to a first embodiment of the invention, seen from the side which is intended to face away from the user during use;
- Figure 2: shows a sanitary towel with a fragrance means according to a second embodiment of the invention, seen from the side which is intended to face away from the user during use;
- Figure 3: shows a sanitary towel with a fragrance means according to a third embodiment of the invention, seen from the side which is intended to face away from the user during use;
- Figure 4: shows a sanitary towel with a fragrance means according to a fourth embodiment of the invention, seen from the side which is intended to face away from the user during use;
- Figure 5: shows a sanitary towel with a fragrance means according to a fifth embodiment of the invention, seen from the side which is intended to face away from the user during use, and
- Figure 6: shows the sanitary towel in Figure 5 with the fragrance means exposed.

### DESCRIPTION OF ILLUSTRATIVE EMBODIMENTS

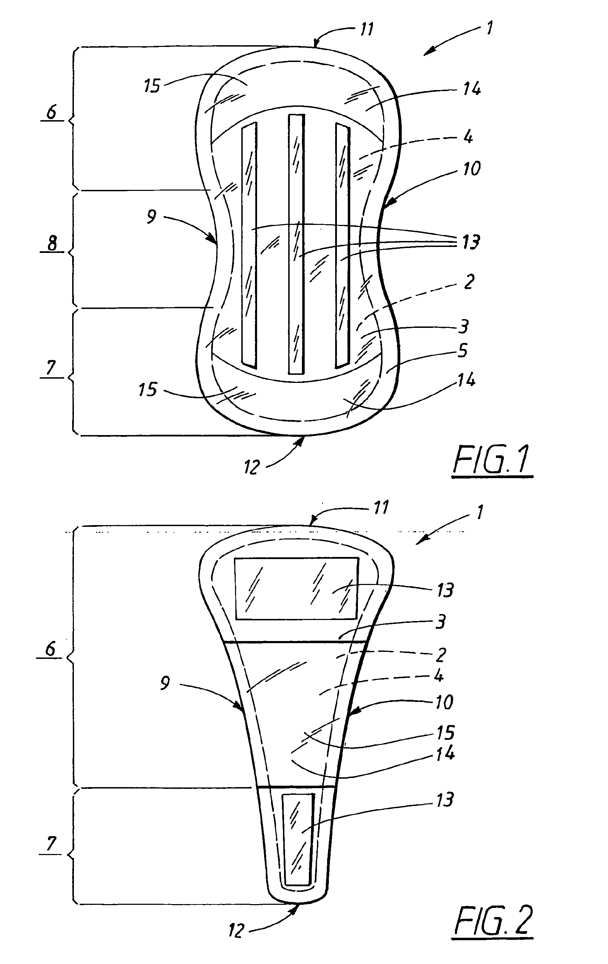

The sanitary towel 1 shown in Figure 1 is shown from the side which is intended to face the underwear of the user when the towel is being worn. The sanitary towel 1 comprises a liquid-permeable surface layer 2, arranged on that side of the sanitary towel 1 which is intended to face the user during use, a liquid-barrier surface layer 3, arranged on that side of the sanitary towel 1 which is intended to face away from the user during use, and an absorbent body 4 enclosed between the two surface layers 2, 3.

The material of the liquid-permeable surface layer 2 can be, for example, a perforated plastic film, a net made of plastic or textile material, a non-woven material or a laminate consisting of, for example, a perforated plastic layer and a non-woven layer. The plastic is usually a thermoplastic, such as polyethylene or polypropylene. Suitable non-woven materials can be made of natural fibres, such as cellulose or cotton, or of synthetic fibres, such as polyethylene, polypropylene, polyester, polyurethane, nylon or regenerated cellulose. It is of course also possible to use non-woven materials made from fibre mixtures.

It is not necessary for the liquid-permeable surface layer 2 actually to consist of a separate material layer. For example, the liquid-permeable covering layer can constitute an integrated part of the absorbent body 4. Absorbent foamed materials, for example, often have a sufficiently good cohesive capacity that a separate liquid-permeable surface layer can be dispensed with. Use can also be made of an absorbent non-woven material, which can be included as a component in an absorbent body and at the same time constitute a liquid-permeable covering layer. Such constructions are not uncommon in very thin towels and panty liners.

The liquid-barrier surface layer 3 consists of a liquid-impermeable material or one which at least resists liquid penetration. Thin, liquid impermeable plastic films are suitable for the purpose. However, it is also possible to use materials which are originally liquid-permeable but have been provided with a coating of plastic, resin or other sealing material. In this way, leakage of liquid from the underside of the absorbent article is prevented. The liquid-blocking surface layer 3 can therefore consist of any material which is skin-friendly and meets the liquid-barrier criterion. Examples of materials suitable as a barrier layer are plastic films, non-woven materials and various types of laminate. Plastic films which can be used are, for example, those made of polyethylene, polypropylene or polyester. Alternatively, the liquid-barrier surface layer 3 can consist of a laminate made of a liquid-impermeable plastic layer facing the absorbent body and a non-woven layer facing the underwear of the user. Such a construction provides a leakproof barrier layer with a textile feel.

In the same way as with the liquid-permeable surface layer 2, it is not necessary for the liquid-barrier surface layer 3 to consist of a separate layer. It is therefore possible to imagine the liquid-barrier surface layer 3 constituting an integrated part of an absorbent material, for example an absorbent foam layer with a liquidimpermeable surface.

The absorbent body 4 can advantageously be made mainly from cellulose fluff pulp. This can be in the form of rolls, bales or sheets which are dry-defibred and converted in fluffed form into a pulp mat, with or without the addition of what are known as superabsorbents, which are polymers with a capacity for absorbing several times their own weight of water or body fluid. Examples of other materials which can be used are various types of natural fibre such as cotton fibres, peat or the like. It is of course also possible to use absorbent synthetic fibres or mixtures of natural fibres and synthetic fibres. The absorbent material can also contain other components, such as liquid-spreading means or binders such as, for example, thermoplastic fibres which have been heat-treated so as to hold short fibres and particles together to form a cohesive unit. It is also possible to use various types of absorbent foamed material in the absorbent body 4.

The two surface layers 2, 3 are interconnected outside the absorbent body 4 and form a projecting edge 5 around the entire periphery of the sanitary towel. The surface layers can be joined together in any suitable way, such as by gluing, sewing or welding using heat or ultrasound.

The sanitary towel 1 is essentially hourglass-shaped and has two end portions 6, 7 and an intermediate narrower crotch portion 8 intended to be arranged between the legs of the user. The sanitary towel 1 also has two inwardly curved side edges 9, 10 and two outwardly curved end edges 11, 12.

The division of the sanitary towel into two end portions 6, 7 and a crotch portion 8 is not to be understood as there being well-defined boundaries between the different portions 6-8 but is primarily intended to facilitate the description of the sanitary towel on the basis of the differences which exist between the different portions 6-8 depending on how they are intended to be positioned in relation to the body of a user. The transition between the different portions 6-8 does not therefore take place at defined transverse lines but rather within transition areas located at a distance of approximately one third of the length of the sanitary towel from the end edges 11, 12 of the sanitary towel. The crotch portion 8 constitutes that part of the sanitary towel 1 which is intended during use to receive and absorb the bulk of the liquid discharged into the sanitary towel during use.

The sanitary towel 1 is provided with an adhesive fastening element 13 in the form of three strands of adhesive which are attached in the crotch portion of the briefs of the user when the towel 1 is worn. Before use, the fastening element 13 is suitably protected in a conventional way by a detachable protective strip which is removed when the adhesive is to be exposed for fastening. The fastening element 13 shown is only an example of the design of an adhesive fastening element. Other patterns and positionings of fastening element are of course possible.

The sanitary towel 1 also has an area 14 in each end portion 6, 7, which area 14 has a coating of fragrance means 15. The fragrance means is in the form of an aromatic substance which is covered by a scrape-off coating. The fragrance means is therefore present in an inactive, non-fragrant form until the user chooses to activate the fragrance means by rubbing or scraping one or both of the areas 14 with fragrance means 15. The positioning of the fragrance means 15 in the end portions 6, 7 of the towel 1 makes it possible easily to reach to activate the fragrance means 15 both before the sanitary towel is fitted in a pair of briefs and during use and after use when the sanitary towel is to be disposed of. The positioning does not interfere with the fastening element 13 either, but the user can activate the fragrance means without risking becoming stuck on the fastening element.

The user can choose to activate the whole or parts of the fragrance means 15 on different occasions, entirely according to personal choice. For users who do not wish to wear fragrance means close to the body, it may nevertheless be desirable for it to be possible to activate the fragrance means in connection with the absorbent article being disposed of so as to conceal bad odour of the used article.

It may be suitable to extend a protective layer for the fastening element 13 so that it also covers the fragrance means 15 in the end portions 6, 7 of the sanitary towel 1. In this way, activating the fragrance means by mistake, for example if it is subjected to friction during storage before use, is avoided. The positioning of the fragrance means 15 in relation to the fastening element 13 means that the same protective layer can be used for both the fastening element 13 and the fragrance means 15.

The sanitary towel 1 shown in Figure 2 is what is known as a string towel, which is shaped so as to fit in a pair of string briefs. String briefs have a very narrow rear portion, and it is therefore important that the sanitary towel 1 is positioned correctly in order to achieve good leakproofness.

The general construction of the string towel shown in Figure 2 is the same as for the sanitary towel in Figure 1. The string towel 1 therefore has a liquid-permeable surface layer 2, a liquid-barrier surface layer 3 and an absorbent body 4 arranged therebetween.

In contrast to the sanitary towel in Figure 1, the string towel in Figure 2 has a trapezoid shape with an essentially triangular front end portion 6 and an essentially rectangular rear end portion 7. The string towel does not have a clearly discernible crotch portion, but that part of the string towel which is intended to be positioned outside the vaginal orifice constitutes a transition area between the front end portion 6 and the rear end portion 7. It will be understood that the string towel is intended to be positioned in the briefs of the user with the front end portion 6 facing towards the abdomen of the user and the rear end portion 7 facing towards the back of the user.

An adhesive fastening element 13 is arranged as two rectangular adhesive surfaces, one on each end portion 6, 7, close to the end edges 11, 12. An area 14 with fragrance means 15 is arranged between the adhesive surfaces of the fastening element. Owing to the fact that the fragrance means 15 is not adhesive, the area 14 between the adhesive surfaces of the fastening element 13 is therefore free of fastening element. This means that this area 14 can move slightly in relation to the briefs of the user when the string towel is being worn, which is an advantage because it increases the capacity of the string towel to adapt to the body of the user. This embodiment is especially advantageous for string towels and other absorbent articles with a centrally located raised portion intended to be fitted against the body of the user.

In the illustrative embodiment shown in Figure 2 as well, it is possible to cover the fastening element 13 and the fragrance means 15 with a single protective layer before use.

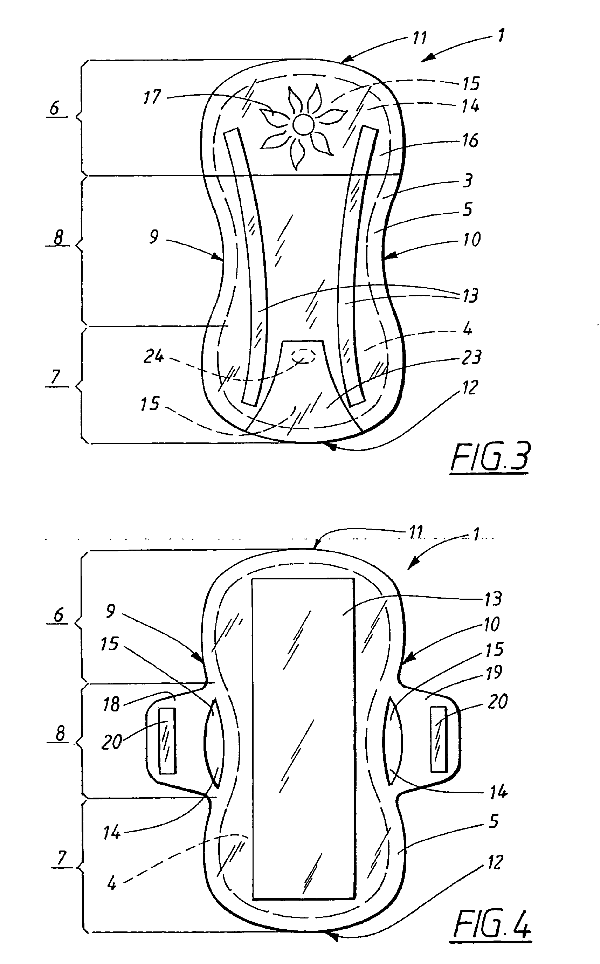

Another sanitary towel 1 is shown in Figure 3. The sanitary towel in Figure 3 differs from that shown in Figure 1 in that the fragrance means 15 is positioned in an area 14 of the liquid-impermeable surface layer 3 which is covered by a separate material layer 16 and located in a first end portion 6 of the sanitary towel. The material layer 16 protects the fragrance means 15 against unintentional activation. The material layer 16 also constitutes activation means for the fragrance means 15. The material layer 16 is attached to the liquid-impermeable surface layer 3 only in the edge join 5. This means that the material layer 16 can be moved slightly in the lateral direction in relation to the liquid-impermeable surface layer 3, for example by the material layer 16 being subjected to a rubbing movement. A suitable material for the material layer 16 is a relatively coarse-fibred non-woven, a net or an embossed layer of plastic film or non-woven which has sufficient friction against the fragrance means 15 in order to activate it. If the material layer 16 is a plastic film, it is suitable if this is perforated, so that the fragrance can be spread to the surrounding environment.

One advantage of the embodiment shown in Figure 3 is that the user can activate the fragrance means 15 without having to come into contact with the aromatic substance in the fragrance means.

The adhesive attachment element 13 is positioned so that it extends in the form of two adhesive strands essentially the whole way along the side edges of the sanitary towel 1, with a portion of each adhesive strand located on the material layer 16 arranged over the fragrance means 15.

The sanitary towel 1 in Figure 3 also has a marking 17 in the form of a flower. The marking is intended to indicate visually for the user where the fragrance means 15 is located, so that activation is effected in the correct place. As an alternative to the flower shown, a different pattern, text or simply a differing colour can of course be used. Visually identifiable markings can of course be used in order to indicate the positioning of the fragrance means 15 for any of the embodiments described herein.

The sanitary towel 1 in Figure 3 also has another fragrance means 15 which comprises a material tab 23 which is attached to the second end portion 7 opposite the first end portion 6. An aromatic substance is arranged in the material tab, for example in the form of microcapsules which are attached to the tab and burst when the material tab is rubbed. The material tab 23 is shown folded in against the liquid-impermeable covering layer 3 and attached to it by a detachable attachment 24, suitably consisting of refastenable adhesive. When the fragrance means 15 is to be activated, the attachment 24 is freed and the material tab 23 is folded out from the liquid-impermeable covering layer 3 so that the material tab 23 becomes accessible for rubbing. The two different fragrance means shown in Figure 3 can of course be used individually.

The sanitary towel 1 shown in Figure 4 is what is known as a winged towel, that is to say a sanitary towel with side flaps 18, 19 which project from the respective side edge 9, 10 of the sanitary towel. The side flaps 18, 19 are usually formed by one or both of the surface layers 2, 3 but can alternatively be separate material pieces which are attached to the side edges 9, 10. In the example shown in Figure 4, the side flaps 18, 19 constitute projections on the edge join 5 and comprise both the liquid-permeable surface layer 2 and the liquid-impermeable surface layer 3.

When the sanitary towel is to be used, it is placed in the briefs of the user, with the attachment element 13 (a rectangular adhesive area in the example shown) attached in the crotch portion of the briefs. The side flaps 18, 19 are then folded around the leg edges of the briefs and are attached on the outside of the crotch portion by means of adhesive fastening elements 20 arranged outermost on the side flaps 18, 19. The sanitary towel 1 shown is attached with the side flaps against the briefs. In alternative embodiments, the side flaps 18, 19 can be designed so that they can be fastened together with one another. Although the fastening element 20 shown is an adhesive fastening element, it is of course possible to use other types of fastening element, for example hook and loop surfaces, mechanical clips, press-studs or the like.

Areas 14 with fragrance means 15 are located on each side flap 18, 19, between the absorbent body 4 of the sanitary towel and the fastening elements of the side flaps 18, 19. In order to ensure that fragrance is emitted when the sanitary towel is being worn as well, the liquid-impermeable surface layer 3 can be perforated within the areas 14 with fragrance means 15. It is also possible to arrange the fragrance means 15 on the inside of the liquid-impermeable surface layer 3, between this and the liquid-permeable surface layer 2.

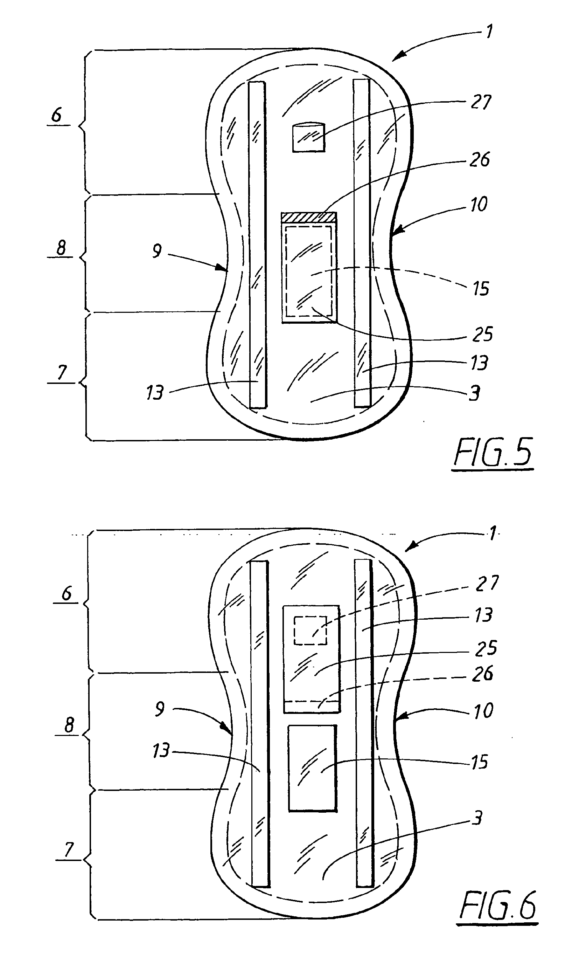

Figures 5 and 6 show a further variant of how a fragrance means 15 can be arranged on an absorbent article. The sanitary towel in Figures 5 and 6 has the same general construction as the sanitary towel in Figure 1, and corresponding components have therefore been provided with the same reference numbers. The sanitary towel is seen from the side which is attached inside the underwear of the user during use. A fastening element 13 in the form of two adhesive strands arranged along the side edges 9, 10 is arranged on the liquid-barrier surface layer 3. Arranged between the adhesive strands of the fastening element 13 is a fragrance means 15 which in Figure 5 is covered by a rectangular protective layer 25 which is attached permanently to the liquid-barrier surface layer 3 along an edge portion 26 of the protective layer 25.

The protective layer 25 is foldable around the attachment and can thus occupy a non-folded protective position as shown in Figure 5 and a folded-away exposure position as shown in Figure 6. In the protective position, the protective layer 25 is attached detachably over the fragrance means 15 by means of, for example, adhesive, welding or the like. In the exposure position, the fragrance means is accessible for activation by rubbing or scraping. Alternatively, the fragrance means can be activated by virtue of the detachment of the protective layer from the fragrance means 15 mechanically breaking a membrane or the shell of microcapsules so that an aromatic substance included in the fragrance means is released. A means 27 for fixing the protective layer in the exposure position is arranged on the liquid-blocking surface layer 3. Such a fixing means can be an adhesive or some type of mechanical attachment element, such as a hook and loop surface. In the exposure position, the protective layer 25 is folded away from the fragrance means 15 and attached to the fixing means. Alternatively, the fastening element 13 of the sanitary towel can of course be utilized as fixing means for the protective layer 25. In such an embodiment, the protective layer must of course be arranged so that it overlaps the fastening element 13 when the protective layer 25 is in the exposure position.

The sanitary towels described here constitute only examples of absorbent articles in which the invention is applicable. It is of course the case that the invention, within the scope of the patent claims, can also be applied to diapers, incontinence pads and panty liners. Furthermore, the various positionings of fragrance means described can be combined with one another.

## Claims

1. An absorbent article such as a sanitary towel, a panty liner, a diaper or an incontinence pad with a longitudinal direction and a transverse direction and with side edges (9, 10) running in the longitudinal direction and end edges (11, 12) running in the transverse direction and comprising a liquid-barrier surface (3), a liquid-permeable surface (2) and an absorbent body (4) between the two surfaces (2, 3), a fragrance means (15) being arranged on one of the surfaces (2, 3) of the article, wherein the fragrance means (15) has an active, fragrant form and an inactive, non-fragrant form and is arranged on the surface (2, 3) of the article in the inactive form and is activatable by mechanical action such as rubbing or scraping, **characterized in that** the positioning of the fragrance means (15) is marked by a visually identifiable indication (17), wherein the fragrance means (15) in its inactive form is covered by a scrape-off odour-impermeable coating.

2. An absorbent article according to Claim 1, **characterized in that** the fragrance means (15) is arranged on the liquid-barrier surface (3).

3. An absorbent article according to Claim 1 or 2, **characterized in that** the article has a first end portion (6) and a second end portion (7), the fragrance means (15) being arranged on the liquid-impermeable surface layer (3) in at least one end portion (6, 7).

4. An absorbent article according to Claim 1, 2 or 3, **characterized in that** an adhesive attachment element (13) is arranged in at least two areas on the liquid-impermeable surface layer (3) in the end portions (6, 7) of the article and **in that** the fragrance means (15) is arranged in an area (14) free of attachment elements between the areas with attachment element (13).

5. An absorbent article according to Claim 2, **characterized in that** the fragrance means (15) is applied to a part surface (14) of the liquid-blocking surface (3) and is covered by a material layer (16) which is attached to the liquid-blocking surface (3) only outside the part surface (14) provided with the fragrance means, the material layer being movable in relation to the fragrance means (15).

6. An absorbent article according to Claim 1 or 2, **characterized in that** the absorbent article comprises another fragrance means (15) which comprises a material tab (23) attached to a surface (2, 3) of the article and comprising mechanically activatable aromatic substance.

7. An absorbent article according to Claim 1 or 2, **characterized in that** the article has side flaps (18, 19) which are arranged at the side edges (9, 10) of the article and have such a shape and size that they can be folded around the leg edges on a pair of briefs and attached in the folded position on the outside of the briefs, the fragrance means (15) being arranged on the side flaps (18, 19).

8. An absorbent article according to Claim 1 or 2, **characterized in that** the fragrance means (15) in the inactive form is covered by a partly detachable protective layer (25).

9. An absorbent article according to Claim 9, **characterized in that** the protective layer (25) is attached to the article along an edge portion (26) of the protective layer (25) and is foldable around the attachment and can thus occupy a non-folded protective position and a folded-away exposure position and **in that** the article comprises means (27) for fixing the protective layer (25) in the exposure position.

## Patentansprüche

1. Saugfähiger Artikel wie z.B. eine Binde, eine Slipeinlage, eine Windel oder ein Inkontinenzschutz mit einer Längsrichtung und einer Querrichtung und mit Seitenkanten (9, 10), die sich in der Längsrichtung erstrecken, und Endkanten (11, 12), die sich in der Querrichtung erstrecken, und umfassend eine Flüssigkeitsbarriereoberfläche (3), eine flüssigkeitsdurchlässige Oberfläche (2) und einen saugfähigen Körper (4) zwischen den zwei Oberflächen (2, 3), wobei ein Duftmittel (15) auf einer der Oberflächen (2, 3) des Artikels angeordnet ist, wobei das Duftmittel (15) eine aktive, duftende Form und eine inaktive, nicht-duftende Form aufweist und auf der Oberfläche (2, 3) des Artikels in der inaktiven Form angeordnet ist und durch einen mechanischen Vorgang wie Reiben oder Kratzen aktiviert werden kann, **dadurch gekennzeichnet, dass** die Positionierung des Duftmittels (15) durch eine visuell identifizierbare Markierung (17) markiert ist, wobei das Duftmittel (15) in seiner inaktiven Form mit einer abkratzbaren geruchundurchlässigen Beschichtung gedeckt ist.

2. Saugfähiger Artikel nach Anspruch 1, **dadurch gekennzeichnet, dass** das Duftmittel (15) auf der Flüssigkeitsbarriereoberfläche (3) angeordnet ist.

3. Saugfähiger Artikel nach Anspruch 1 oder 2, **dadurch gekennzeichnet,** das der Artikel einen ersten Endteil (6) und einen zweiten Endteil (7) aufweist, wobei das Duftmittel (15) auf der flüssigkeitsundurchlässigen Oberflächenschicht (3) in mindestens einem Endteil (6, 7) angeordnet ist.

4. Saugfähiger Artikel nach Anspruch 1, 2 oder 3, **dadurch gekennzeichnet, dass** ein klebendes Anhaftungselement (13) in mindestens zwei Bereichen auf der flüssigkeitsundurchlässigen Oberflächenschicht (3) in den Endteilen (6, 7) des Artikels angeordnet ist, und dass das Duftmittel (15) in einem Bereich (14) ohne Anhaftungselemente zwischen den Bereichen mit Anhaftungselement (13) angeordnet ist.

5. Saugfähiger Artikel nach Anspruch 2, **dadurch gekennzeichnet, dass** das Duftmittel (15) auf einer Teiloberfläche (14) der flüssigkeitsblockierenden Oberfläche (3) aufgebracht ist und mit einer Materialschicht (16) gedeckt ist, die nur außerhalb der mit dem Duftmittel versehenen Teiloberfläche (14) auf der flüssigkeitsblockierenden Oberfläche (3) befestigt ist, wobei die Materialschicht im Verhältnis zum Duftmittel (15) bewegbar ist.

6. Saugfähiger Artikel nach Anspruch 1 oder 2, **dadurch gekennzeichnet, dass** der saugfähige Artikel ein anderes Duftmittel (15) umfasst, das eine Materiallasche (23) umfasst, die mit einer Oberfläche (2, 3) des Artikels verbunden ist und einen mechanisch aktivierbaren aromatischen Stoff umfasst.

7. Saugfähiger Artikel nach Anspruch 1 oder 2, **dadurch gekennzeichnet, dass** der Artikel Seitenklappen (18, 19) aufweist, die an den Seitenkanten (9, 10) des Artikels angeordnet sind und eine solche Form und Größe aufweisen, dass sie um die Beinkanten einer Unterhose herumgefaltet und in der gefalteten Position an der Außenseite der Unterhose befestigt werden können, wobei das Duftmittel (15) auf den Seitenklappen (18, 19) angeordnet ist.

8. Saugfähiger Artikel nach Anspruch 1 oder 2, **dadurch gekennzeichnet, dass** das Duftmittel (15) in der inaktiven Form mit einer zum Teil abtrennbaren Schutzschicht (25) gedeckt ist.

9. Saugfähiger Artikel nach Anspruch 9, **dadurch gekennzeichnet, dass** die Schutzschicht (25) entlang einem Kantenteil (26) der Schutzschicht (25) an dem Artikel befestigt ist und um die Befestigung herumgefaltet werden kann und somit eine nicht-gefaltete Schutzposition und eine weggefaltete Freilegungsposition einnehmen kann, und dass der Artikel Mittel (27) zur Befestigung der Schutzschicht (25) in den Freilegungsposition umfasst.

## Revendications

1. Article absorbant tel qu'une serviette hygiénique, un protège-slip, une couche ou une serviette pour incontinence avec une direction longitudinale et une direction transversale et avec des bords latéraux (9, 10) s'étendant dans la direction longitudinale et des bordures latérales (11, 12) s'étendant dans la direction transversale et comprenant une surface de barrière aux liquides (3), une surface perméable aux liquides (2) et un corps absorbant (4) entre les deux surfaces (2, 3), un système de parfum (15) étant arrangé sur l'une des surfaces (2, 3) de l'article, le système de parfum (15) présentant une forme de parfum actif et une forme non parfumée inactive et étant arrangé sur la surface (2, 3) de l'article dans la forme inactive, l'activation se faisant par action mécanique du type frottement ou grattage, **caractérisé en ce que** le positionnement du système de parfum (15) est marqué par une indication visuellement identifiable (17), le système de parfum (15) dans sa forme inactive étant recouvert d'un revêtement d'arrachement imperméable aux odeurs.

2. Article absorbant selon la revendication 1, **caractérisé en ce que** le système de parfum (15) est disposé sur la surface de barrière aux liquides (3).

3. Article absorbant selon la revendication 1 ou 2, **caractérisé en ce que** l'article présente une première partie d'extrémité (6) et une deuxième partie d'extrémité (7), le système de parfum (15) étant disposé sur la couche de surface imperméable aux liquides (3) dans au moins une partie d'extrémité (6, 7).

4. Article absorbant selon la revendication 1, 2 ou 3, **caractérisé en ce qu**'un élément de fixation adhésive (13) est pourvu dans au moins deux zones sur la couche de surface imperméable aux liquides (3) dans les parties d'extrémité (6, 7) de l'article, et **en ce que** le système de parfum (15) est disposé dans une zone (14) exempte d'éléments de fixation entre les zones avec un élément de fixation (13).

5. Article absorbant selon la revendication 2, **caractérisé en ce que** le système de parfum (15) est appliqué sur une surface de partie (14) de la surface de barrière aux liquides (3) et est recouvert par une couche de matériau (16) qui est fixée à la surface de barrière aux liquides (3) uniquement à l'extérieur de la surface de partie (14) pourvue du système de parfum, la couche de matériau pouvant être déplacée par rapport au système de parfum (15).

6. Article absorbant selon la revendication 1 ou 2, **caractérisé en ce que** l'article absorbant comprend un autre système de parfum (15) qui comprend une patte de matériau (23) fixée à une surface (2, 3) de l'article et comprenant une substance aromatique et mécaniquement actionnable.

7. Article absorbant selon la revendication 1 ou 2, **caractérisé en ce que** l'article présente des rabats latéraux (18, 19) qui sont disposés aux bords latéraux (9, 10) de l'article et présentent une telle forme et taille qu'ils peuvent être pliés autour des bords de jambe sur une paire de slip et fixés dans la position pliée sur l'extérieur du slip, le système de parfum (15) étant disposé sur les rabats latéraux (18, 19).

8. Article absorbant selon la revendication 1 ou 2, **caractérisé en ce que** le système de parfum (15) dans sa forme inactive est recouvert par une couche protectrice et partiellement détachable (25).

9. Article absorbant selon la revendication 9, **caractérisé en ce que** la couche protectrice (25) est fixée à l'article le long d'une portion latérale (26) de la couche protectrice (25) et est pliable autour de la fixation et peut ainsi occuper une position protectrice non pliée et une position d'exposition repliée, et **en ce que** l'article comprend des moyens (27) pour fixer la couche protectrice (25) dans la position d'exposition.
